# NOUVEAU FASCICULE DE BREVET EUROPEEN

(11) **EP 0 326 829 B2**
(45) Date de publication et mention de la décision concernant l'opposition: **06.12.2000**
(45) Mention de la délivrance du brevet: 27.04.1994
(21) Numéro de dépôt: 89100575.3
(22) Date de dépôt: 13.01.1989
(51) Int. Cl.: C09K 15/04, C11B 5/00, A23D 7/06, A23D 9/06, A61K 7/00, C09K 15/32

(54) **Mélange antioxydant synergique**
Synergetische Antioxydationsmischung
Synergetic antioxidant mixture

(30) Priorité: 03.02.1988 CH 37488
(43) Date de publication de la demande: 09.08.1989
(73) Titulaire: SOCIETE DES PRODUITS NESTLE S.A., 1800 Vevey (CH)
(72) Inventeur: Löliger, Jürg, CH-1802 Corseaux (CH); Saucy, Françoise, CH-1807 Blonay (CH)

(56) Documents cités:
- EP-A- 0 176 772
- EP-A- 0 239 086
- EP-A- 0 280 606
- DE-B- 1 492 578
- US-A- 2 432 698
- DERWENT FILE SUPPLIER WPIL, 1985, AN no. 85-034347[06], Derwent Publications Ltd, Londres, GB; & JP-A-59 227 876
- PATENT ABSTRACTS OF JAPAN, vol. 8, no. 134 (C-230)[1571], 21 juin 1984, page 94 C 230; & JP-A-59 45 860
- DERWENT FILE SUPPLIER WPIL, 1982, AN no. 82-15158E [08], Derwent Publications Ltd, Londres, GB; & SU-A-822 824
- Aoyama, Minoru et al., Yukagaku (1985), 34 (6), pp. 470-475
- G. Pongracz, Intern. J. Vit. Nutr. Res., 43 (1978)

## Description

L'invention concerne une matière grasse ou un produit alimentaire ou cosmétique protégé de l'oxydation au moyen d'un mélange antioxydant synergique.

La tendance actuelle en matière d'antioxydants, en particulier à usage alimentaire, consiste à privilégier les composés naturels qui possédent une activité antioxydante.

Les esters de l'acide ascorbique avec les acides gras saturés, en particulier le palmitate (AP) et le stéarate d'ascorbyle (AS), sont connus comme antioxydants des lipides. C'est également le cas des tocophérols (TL) dont on a observé l'activité antioxydante dans les graisses animales n'en contenant pas naturellement. On sait aussi qu'un mélange de AP et TL est plus actif que chacun de ces composés seul.

On a montré également, par exemple G.Pongraz dans In.J.Vit.Nutr.Res. 43 (1973), que la lécithine (LC), bien que ne possédant pas d'activité antioxydante propre, permettait d'augmenter fortement l'activité du mélange AP et TL dans l'huile de beurre et dans l'huile de tournesol.

Cette observation est confirmée dans la demande de brevet japonais publiée No 80.069688 relative au mélange AS, TL et LC dans l'huile de carthame et dans le saindoux.

Dans ces mélanges connus, l'acide ascorbique (AA) n'est pas mis en oeuvre comme tel mais sous forme d'ester (AP, AS) pour la simple raison qu'il n'est pas liposoluble. D'après G.Pongraz dans l'article précédent, AP et AS sont équivalents du point de vue de leur activité antioxydante dans les lipides et la préférence est donnée à AP à cause de sa liposolubilité légèrement meilleure.

US-A-2432698 concerne la protection contre l'oxydation des vitamines liposolubles particulièrement sensibles à l'oxydation A et D en dispersion concentrée dans un support solide ou d'une huile de foie de poisson contenant ces vitamines liposolubles.

Le mélange antioxydant utilisé comprend comme éléments essentiels:
- La niacine ou l'un de ses dérivés et
- Une matière végétale contenant des antioxydants naturels constituée de farine de graines, noyaux ou légumineuses ou d'extraits, concentrats ou fractions de telles matières.

Lorsqu'il est question de protéger une graisse, il est précisé qu'il est nécessaire d'ajouter un solvant mutuel des composés antioxydants hydrosolubles (ici la niacinamide) et liposolubles du mélange pour l'incorporer dans la graisse, sans que le solvant ne soit ensuite éliminé et c'est pour cette raison que dans ce cas la préférence est donnée aux composants antioxydants liposolubles.

Nous avons trouvé de manière surprenante que l'AA, lorsqu'il est utilisé en présence de TL et de phospholipide, a une activité antioxydante bien supérieure à celle de ses esters dans les systèmes anhydres, en particulier les lipides.

C'est ainsi que la matière grasse, le produit alimentaire ou cosmétique contenant une matière grasse protégé de l'oxydation selon l'invention comprend, par rapport au poids de la matière grasse, 0,55 à 2,3 % d'un mélange antioxydant synergique:
constitué de tocophérol, d'acide ascorbique et d'un phospholipide,
dans les proportions de 2,5 à 10% de tocophérol et 2,5 à 20% d'acide ascorbique par rapport au poids de phospholipide
et forme une dispersion stable de l'acide ascorbique dans un milieu anhydre lipophile.

Comme tocophérol, on peut mettre en oeuvre l'alpha- , le béta- , le gamma-, le delta-tocophérol ou des mélanges de ces tocophérols, par exemple un mélange naturel provenant d'une huile végétale, par exemple de l'huile de soja, de l'huile de germe de blé, de l'huile de graines de coton.

Les phospholipides mis en oeuvre peuvent être d'origine animale ou végétale, du lait, de l'oeuf, du soja. Ce sont de préférence les lécithines, par exemple les lécithines commerciales, les lécithines purifiées, les fractions de lécithines de soja. Le phospholipide permet de former une dispersion stable de l'AA dans un produit anhydre, par exemple une matière grasse ou un aliment contenant une matière grasse ou encore un produit cosmétique contenant une matière grasse. On préfère utiliser les lécithines de soja ou leurs fractions qui sont abondantes et économiques.

Le mélange antioxydant selon l'invention contient de préférence environ 5% de TL et 5 à 20% d'AA, par rapport au poids de phospholipide. Si l'on met en oeuvre moins de 0,55% en poids de mélange, on risque de ne pas obtenir l'effet synergique souhaité en raison de la trop faible quantité phospholipide ou d'antioxydant. Si l'on utilise plus de 2,3% en poids de mélange, on court le risque d'induire des effets secondaires indésirables tels que des variations de goût ou d'odeur ou la formation de mousse, par exemple.

L'invention concerne en outre un procédé de préparation du mélange antioxydant synergique constitué de tocophérol, d'acide ascorbique et d'un phospholipide tel que défini précédemment, caractérisé par le fait que l'on mélange le phospholipide et le tocophérol sous agitation à température inférieure ou égale à 60°C, que l'on ajoute progressivement à ce prémélange l'acide ascorbique dissout dans un solvant polaire en présence d'un gaz inerte, puis que l'on élimine le solvant à une température inférieure ou égale à 60°C.

Selon un mode de réalisation avantageux du procédé selon l'invention, on prépare le mélange par mise en présence de LC et du TC sous agitation, à température inférieure ou égale à 60°C, avec barbottage d'un gaz inerte, par exemple l'azote. A ce pré-mélange, on ajoute ensuite progressivement l'AA dissout dans un solvant polaire, à bas point d'ébullition, par exemple l'éthanol, puis on élimine le solvant à une température ≦ 60°C, sous un léger vide. Le mélange obtenu se présente sous forme d'un liquide transparent et visqueux. Ce mélange peut être utilisé de manière différée, par exemple en l'incorporant dans une matière grasse à protéger, de préférence à chaud, le mélange étant à 60°C environ, sous agitation vigoureuse.

L'invention concerne enfin un procédé de protection d'une matière grasse de l'oxydation par incorporation d'acide ascorbique, de phospholipide et de tocophérol, caractérisé par le fait que l'on ajoute le phospholipide à la matière grasse, que l'on dissout le tocophérol et l'acide ascorbique dans un solvant polaire, que l'on mélange la solution d'acide ascorbique et de tocophérol avec la matière grasse contenant le phospholipide, puis que l'on élimine le solvant à une température inférieure ou égale à 60°C en présence d'un gaz inerte, de sorte que le tocophérol représente 2,5 à 10% en poids de phospholipide et l'acide ascorbique, 2,5 à 20% en poids du phospholipide et que le tocophérol, l'acide ascorbique et le phospholipide représentent 0,55 à 2,3% en poids de la matière grasse.

Le solvant polaire utilisé est de préférence l'alcool éthylique.

Les matières grasses à protéger selon l'invention sont de préférence les plus sensibles à l'oxydation, par exemple celles qui sont riches en acides gras insaturés, particulièrement polyinsaturés. On peut citer les huiles végétales, par exemple de germes de blé, de pépins de raisins, de maïs, de soja, de carthame, d'olive, d'onagre, de bourrache, spécialement l'huile de pépins de cassis. Comme matière grasse animale sensible à l'oxydation on peut citer la graisse de poule, l'huile de beurre, les huiles d'animaux marins, en particulier de poisson.

Les aliments et produits cosmétiques à protéger sont de préférence ceux qui contiennent de tels corps gras.

Les exemples suivants illustrent l'invention. Dans ceux-ci, les pourcentages et parties sont en poids, sauf indication contraire.

### Exemple 1-6

On évalue le pouvoir antioxydant des mélanges selon l'invention dans la protection de l'huile de poisson contre l'oxydation au moyen du test d'oxydation accélérée Fira-Astell®.

### Préparation des échantillons

On prépare des échantillons de 4 g d'huile stabilisée de la manière suivante: on dissout les antioxydants dans de l'éthanol absolu à raison de 125 mg / 25 ml et on mélange la solution à l'huile dans laquelle on a préalablement ajouté, le cas échéant, la lécithine. Selon les concentrations mises en oeuvre, on utilise 0,5 à 1,5 ml d'éthanol. On élimine ensuite l'éthanol par évaporation à 60°C pendant 2 h en balayant l'échantillon avec de l'azote.

### Test d'oxydation

On place l'échantillon dans un réacteur en verre fermé hermétiquement pourvu d'un agitateur magnétique. Le réacteur est lui-même placé dans un bain d'huile à la température choisie. L'atmosphère dans le réacteur est l'air. L'espace de tête communique par un tube souple avec un manomètre différentiel relié à un enregistreur. Lorsque l'oxydation progresse, la quantité d'oxygène absorbée est indiquée par la différence de pression observée. On détermine le temps d'induction graphiquement à partir de la courbe transcrite de la pression en fonction du temps par intersection de la tangente à la courbe avec l'axe des temps.

Les résultats obtenus sont indiqués dans le tableau I ci-après, dans lequel on donne à titre comparatif les temps d'induction obtenus sans additif (Co), par les différents additifs seuls (C1 à C3) ou deux par deux (C4 à C13).

**Tableau I**

| Exemples | Additif antioxydant, par rapport à l'huile | | | Temps d'induction¹ (h) à | | |
|---|---|---|---|---|---|---|
| | TL(ppm) | AA(ppm) | LC(%) | 60°C | 80°C | 100°C |
| 1 | 250 | 250 | 1 | - | 2,7 | - |
| 2 | 250 | 500 | 1 | - | 9,1 | - |
| 3 | 500 | 500 | 1 | - | 12,8 | 3 |
| 4 | 500 | 1000 | 1 | - | 23,2 | 5,2 |
| 5 | 1000 | 500 | 1 | - | 11,2 | - |
| 6 | 500 | 2000 | 1 | - | 18 | - |

| Comparaisons: | | | | | | |
|---|---|---|---|---|---|---|
| C0 | - | - | - | 7 | 0,5 | 0,1 |
| C1 | 500 | - | - | 12 | - | - |
| C2 | - | 1000 | - | 11,5 | - | - |
| C3 | - | - | 1 | 10 | 1,6 | - |
| C4 | 500 | 250 | - | 11,7 | - | - |
| C5 | 500 | 500 | - | 17 | 2,9 | - |
| C6 | 250 | 500 | - | - | 2,5 | - |
| C7 | 250 | 1000 | - | - | 2,9 | - |
| C8 | 500 | 1000 | - | - | 2,5 | - |
| C9 | 1000 | 500 | - | - | 3,6 | - |
| C10 | 2000 | 2000 | - | - | 2,7 | - |
| C11 | 500 | - | 1 | - | - | 2,2 |
| C12 | 1000 | - | 1 | - | 1,2 | - |
| C13 | - | 500 | 1 | - | - | 1,1 |
| Légende: ppm = partie par million LC = lécithine de soja-purifiée, Topcithin®. | | | | | | |

| | | | | | | |
|---|---|---|---|---|---|---|
| 1 La comparaison des résultats obtenus pour le temps d'induction aux différentes températures indiquées peut être faite en utilisant un facteur approximatif de 4 pour les valeurs à 100°C par rapport aux valeurs à 80°C. | | | | | | |

Il ressort du tableau I précédent que l'adjonction de mélange ternaire TL, AA et LC a un effet tel que l'huile de poisson reste stable environ 5 à 26 fois plus longtemps que l'huile sans additif (1 et 4 comparés à C0) et environ 12 fois plus longtemps que l'huile additionnée de l'un ou l'autre des antioxydants à des quantités comparables (4 comparé à C1-C3).

L'effet antioxydant du mélange ternaire est également bien supérieur, environ 3 à 9 fois, à ceux des mélanges binaires (2 à 5 comparés à C5, C6, C8 et C9).

Il y a donc un effet de synergie des différents additifs.

### Exemple 7

On compare les temps d'induction de l'huile de poisson contenant les mélanges ternaires des exemples 3 et 4 avec ceux obtenus pour l'ascorbyl palmitate (AP) seul, en mélanges binaires ou ternaires avec TL et LC en utilisant le test d'oxydation Fira-Astell® décrit précédemment.

Les résultats sont indiqués dans le tableau II ci-après.

**Tableau II**

| Comparaison | Additif antioxydant | | | Temps d'induction (h) à | | |
|---|---|---|---|---|---|---|
| | TL(ppm) | LC(%) | AA¹(ppm) | 60°C | 80°C | 100°C |
| C14 | - | - | 500 | 8,1 | 1,8 | - |
| C15 | - | 1 | 500 | - | - | 0,2 |
| C16 | - | 1 | 1000 | - | - | 1,3 |
| C17 | 500 | 1 | 500 | - | 4,1 | 1 |
| C18 | 500 | 1 | 1000 | - | 8,1 | 2,2 |
| selon l'exemple 3 | | | | - | 12,8 | 3 |
| selon l'exemple 4 | | | | - | 23,2 | 5,2 |

| | | | | | | |
|---|---|---|---|---|---|---|
| Légende: 1 La teneur indiquée est rapportée à l'acide ascorbique fourni sous forme d'ascorbyl palmitate. LC = Topcithin® | | | | | | |

La comparaison des temps d'induction des systèmes ternaires C17 et exemple 3, respectivement C18 et exemple 4 montre une augmentation de l'effet antioxydant de 2,3 à 3,1 fois par remplacement de l'ascorbyl palmitate par l'acide ascorbique. Ceci est d'autant plus inattendu que l'acide ascorbique est totalement insoluble dans les huiles.

### Exemples 8 - 12

En utilisant le test d'oxydation Fira-Astell® décrit précédemment,on détermine les temps d'induction à 100°C de l'huile de poisson stabilisée par des mélanges antioxydants contenant 500 ppm de TL, 1000 ppm de AA et 1% de différentes lécithines. Les résultats sont rassemblés dans le tableau III ci-après:

**Tableau III**

| Exemple | Emulsifiant | Temps d'induction (h) |
|---|---|---|
| 8 | Azol®: fraction de lécithine de soja mélangée à environ 60% de triglycérides | 6 |
| 9 | Centrophase®: mélange à quantités sensiblement égales de lécithine de soja et de triglycérides | 2,7 |
| 10 | M-C-Thin®: mélange de phospholipides de soja: phosphatidylcholine, phosphatidyléthanolamine phosphatide méso-inositol | 4,2 |
| 11 | Métharin®: mélange de phospholipides de soja et de mono-, di- et triglycérides | 1,5 |
| 12 | Topcithin®: lécithine de soja raffinée pauvre en métaux lourds | 5,2 |

### Exemple 13

En utilisant le test d'oxydation accélérée Rancimat®, on détermine les temps d'induction de la graisse de poule stabilisée avec les additifs antioxydants.

Le test Rancimat diffère du test Fira-Astell® précédent en ce que l'on fait passer de l'air dans un tube de réaction contenant un échantillon de 5 g de matière grasse à 100°C et l'on mesure la conductivité des produits secondaires volatils formés en cours d'oxydation et entraînés avec le courant d'air. On détermine le temps d'induction graphiquement à partir de la courbe enregistrée de la conductivité en fonction du temps par intersection de la tangente à la courbe avec l'axe des temps.

Les résultats sont indiqués dans le tableau IV ci après:

**Tableau IV**

| | Additif antioxydant | | | Temps d'induction (h) |
|---|---|---|---|---|
| | TL(ppm) | AA(ppm) | LC(%) | |
| | 1000 | 500 | 1 | 47,9 |
| Comparaison | 1000 | - | - | 13 |
| Comparaison | - | 500 | - | 7,4 |
| Sans additif | | | | 5 |
| Légende: LC = Topcithin® | | | | |

L'effet antioxydant surprenant du mélange ternaire est confirmé dans la graisse de poule.

### Exemple 14

On détermine les temps d'induction à 100°C de l'huile de maïs stabilisée avec différents additifs antioxydants en utilisant le test d'oxydation Rancimat® décrit précédemment. L'huile de maïs contient déjà naturellement environ 310 ppm de delta-tocophérol (TL).

Les résultats sont rassemblés dans le tableau V ci-après:

**Tableau V**

| | Additif antioxydant | | Temps d'induction (h) |
|---|---|---|---|
| | AA(ppm) | LC(%) | |
| Comparaison | 250 | 0,5 | 34,6 |
| | 250 | - | 20,8 |
| Sans antioxydant autre que TL présent naturellement | | | 10 |
| Légende: LC = Topcithin® | | | |

On voit que l'effet antioxydant surprenant du mélange ternaire est confirmé dans le cas de l'huile de maïs où l'un des antioxydants du mélange, à savoir le tocophérol, est déjà présent naturellement.

### Exemples 15-17

### (Exemple 15)

On chauffe 1000 g de LC (Topcithin®), et 60 g de TL à 60°C avec barbottage d'azote. Sous agitation mécanique, on y ajoute progressivement 100 g d'AA dissout dans 2,5 l d'éthanol absolu en 5 h. On évapore ensuite l'éthanol à 60°C sous léger vide jusqu'à ce que le mélange garde un poids constant. A la fin de l'opération, le mélange devient complètement transparent.

On chauffe 99 kg d'huile de pépins de cassis à 90°C dans une cuve fermée à double manteau sous azote. On y ajoute ensuite le mélange antioxydant précédent chauffé à 60°C en brassant vigoureusement, puis on refroidit l'huile stabilisée à la température ambiante en 20 min.

### (Exemples 15-17)

On détermine les temps d'induction à 100°C de l'huile stabilisée avec différentes quantités d'additifs oxydants et sans additif à l'aide du test d'oxydation Fira-Astell® décrit précédemment.

Les résultats sont indiqués dans le tableau VI ci-après:

**Tableau VI**

| Exemple | Additif antioxydant | | | Temps d'induction (h) |
|---|---|---|---|---|
| | LC(%) | TL(ppm) | AA(ppm) | |
| 15 | 1 | 600 | 1000 | 23 |
| 16 | 0,5 | 250 | 500 | 14,2 |
| 17 | 2 | 1000 | 2000 | 30 |
| Comparaison | sans additif | | | 3,5 |

L'huile de pépins de cassis non stabilisée montre un temps d'induction environ 4 à 9 fois inférieur à ceux obtenus par addition des mélanges antioxydants LC, TL et AA.

## Revendications

1. Matière grasse ou produit alimentaire ou cosmétique contenant une matière grasse protégé de l'oxydation comprenant, par rapport au poids de la matière grasse, 0,55 à 2,3% d'un mélange antioxydant synergique:
- constitué de tocophérol, d'acide ascorbique et d'un phospholipide,
- dans les proportions de 2,5 à 10% de tocopherol et de 2,5 à 20% d'acide ascorbique par rapport au poids de phospholipide,
- et formant une dispersion stable de l'acide ascorbique dans un milieu anhydre lipophile

2. Matière grasse ou produit alimentaire ou cosmétique contenant une matière grasse protégé de l'oxydation selon la revendication 1, caractérisé par le fait que le mélange antioxydant synergique contient environ 5% de tocophérol et 5 à 20% d'acide ascorbique par rapport au poids de phospholipide.

3. Matière grasse ou produit alimentaire ou cosmétique contenant une matière grasse protégé de l'oxydation selon la revendication 1, caractérisé par le fait que le phospholipide du mélange antioxydant synergique est la lécithine de soja, la lécithine de jaune d'oeuf ou leurs fractions.

4. Procédé de préparation du mélange antioxydant constitué de tocophérol, d'acide ascorbique et d'un phospholipide, tel que défini dans la revendication 1, caractérisé par le fait que l'on mélange le phospholipide et le tocophérol sous agitation à température inférieure ou égale à 60°C, que l'on ajoute progressivement à ce prémélange l'acide ascorbique dissout dans un solvant polaire en présence d'un gaz inerte puis que l'on élimine le solvant à une température inférieure ou égale à 60°C.

5. Procédé de protection d'une matière grasse de l'oxydation par incorporation d'acide ascorbique, de phospholipide et de tocophérol, caractérisé par le fait que l'on ajoute le phospholipide à la matière grasse, que l'on dissout le tocophérol et l'acide ascorbique dans un solvant polaire, que l'on mélange la solution d'acide ascorbique et de tocophérol avec la matière grasse contenant le phospholipide, puis que l'on élimine le solvant à une température inférieure ou égale à 60°C en présence d'un gaz inerte, de sorte que le tocophérol représente 2,5 à 10% en poids du phospholipide et l'acide ascorbique 2,5 à 20% en poids du phospholipide et que le tocophérol, l'acide ascorbique et le phospholipide représentent 0,55 à 2,3% en poids de la matière grasse.

6. Matière grasse ou produit alimentaire ou cosmétique contenant une telle matière grasse protégé de l'oxydation par la mise en oeuvre du procédé selon la revendication 5.

7. Matière grasse animale, notamment huile d'animaux marins protégée de l'oxydation par la mise en oeuvre du procédé selon la revendication 5.

8. Huile végétale, notamment de pépins de cassis protégée de l'oxydation par la mise en oeuvre du procédé selon la revendication 5.

## Claims

1. Fat or food product or cosmetic containing a fat protected from oxidation comprising, based on the weight of fat, 0.55 to 2.3 % of a synergetic antioxidant mixture:
- consisting of tocopherol, ascorbic acid and a phospholipid,
- in the proportions of 2.5 to 10 % tocopherol and 2.5 % to 20 % ascorbic acid based on the weight of phospholipid,
- and forming a stable dispersion of ascorbic acid in a lipophilic anhydrous medium.

2. Fat or food product or cosmetic containing a fat protected from oxidation according to claim 1, characterized in that the synergetic antioxidant mixture contains approximately 5 % tocopherol and 5 to 20 % ascorbic acid based on the phospholipid weight.

3. Fat or food product or cosmetic containing a fat protected from oxidation according to claim 1, characterized in that the phospholipid of the synergetic antioxidant mixture is soya lecithin, egg yolk lecithin or fractions thereof.

4. Method for preparing the antioxidant mixture consisting of tocopherol, ascorbic acid and a phospholipid, such as defined in claim 1, characterized in that the phospholipid and tocopherol are mixed with stirring at a temperature below or equal to 60°C, that ascorbic acid dissolved in a polar solvent is added progressively to this premix in the presence of an inert gas and the solvent is then removed at a temperature below or equal to 60°C.

5. Method for protecting a fat from oxidation by incorporating ascorbic acid, phospholipid and tocopherol, characterized in that the phospholipid is added to the fat, that the tocopherol and ascorbic acid are dissolved in a polar solvent, that the solution of ascorbic acid and tocopherol is mixed with the fat containing the phospholipid, that the solvent is then removed at a temperature below or equal to 60°C in the presence of an inert gas, so that the tocopherol represents 2.5 to 10 % by weight of the phospholipid and the ascorbic acid 2.5 to 20 % by weight of the phospholipid and that the tocopherol, the ascorbic acid and the phospholipid represent 0.55 to 2.3 % by weight of the fat.

6. Fat or food product or cosmetic containing such a fat protected from oxidation by the implementation of the method according to claim 5.

7. Animal fat, in particular the oil of marine animals protected from oxidation by the implementation of the method according to claim 5.

8. Vegetable oil, in particular of black currant seeds protected from oxidation by the implementation of the method according to claim 5.

## Patentansprüche

1. Fett oder Nahrungsmittel- oder Kosmetikprodukt, das ein gegen Oxidation geschütztes Fett enthält, das, bezogen auf das Gewicht des Fetts, 0,55 bis 2,3% eines synergetischen Antioxidanziengemischs aufweist,
- das zusammengesetzt ist aus Tocopherol, Ascorbinsäure und einem Phospholipid,
- in Anteilen von 2,5 bis 10% Tocopherol und von 2,5 bis 20% Ascorbinsäure, bezogen auf das Gewicht des Phospholipids,
- und eine stabile Dispersion der Ascorbinsäure in einem wasserfreien lipophilen Milieu bildet.

2. Fett oder Nahrungsmittel- oder Kosmetikprodukt, das ein gegen Oxidation geschütztes Fett enthält, nach Anspruch 1, dadurch gekennzeichnet, daß die synergetische Antioxidanzienmischung etwa 5% Tocopherol und 5 bis 20% Ascorbinsäure, bezogen auf das Gewicht des Phospholipids, enthält.

3. Fett oder Nahrungsmittel- oder Kosmetikprodukt, das ein gegen Oxidation geschütztes Fett enthält, nach Anspruch 1, dadurch gekennzeichnet, daß das Phospholipid der synergetischen Antioxidanzienmischung Sojalecithin, Eigelblecithin oder deren Fraktionen ist.

4. Verfahren zur Herstellung eines Antioxidanziengemischs, das aus Tocopherol, Ascorbinsäure und einem Phospholipid besteht, wie es in Anspruch 1 definiert ist, dadurch gekennzeichnet, daß man das Phospholipid und das Tocopherol unter Rühren bei einer Temperatur unterhalb von oder gleich 60°C mischt, daß man der Vormischung allmählich die Ascorbinsäure zugibt, die in einem polaren Lösungsmittel gelöst ist, in Gegenwart eines Inertgases, wonach man das Lösungsmittel bei einer Temperatur unterhalb von oder gleich 60°C entfernt.

5. Verfahren zum Schützen eines Fetts gegen Oxidation durch Einarbeitung von Ascorbinsäure, Phospholipid und Tocopherol, dadurch gekennzeichnet, daß man das Phospholipid dem Fett zugibt, daß man das Tocopherol und die Ascorbinsäure in einem polaren Lösungsmittel auflöst, daß man die Lösung von Ascorbinsäure und Tocopherol mit dem Fett, das das Phospholipid enthält, vermischt, wonach man das Lösungsmittel bei einer Temperatur unterhalb von oder gleich 60°C in Gegenwart eines Inertgases entfernt, und zwar so, daß das Tocopherol 2,5 bis 10% des Gewichts des Phospholipids und die Ascorbinsäure 2,5 bis 20% des Gewichts des Phospholipids ausmachen und das Tocopherol, die Ascorbinsäure und das Phospholipid 0,55 bis 2,3% des Gewichts des Fetts ausmachen.

6. Fett oder Nahrungsmittel- oder Kosmetikprodukt, das ein Fett enthält, das durch die Anwendung des Verfahrens nach Anspruch 5 gegen Oxidation geschützt ist.

7. Tierfett, insbesondere ein Seetieröl, das durch die Anwendung des Verfahrens nach Anspruch 5 gegen Oxidation geschützt ist.

8. Pflanzenöl, insbesondere Johannisbeerenkernöl, das durch die Anwendung des Verfahrens nach Anspruch 5 gegen Oxidation geschützt ist.
